(19) Europäisches Patentamt
European Patent Office
Office européen des brevets

(11) **EP 2 036 041 B1**

(12) **EUROPEAN PATENT SPECIFICATION**

(45) Date of publication and mention
of the grant of the patent:
**25.08.2010 Bulletin 2010/34**

(51) Int Cl.:
***G06T 7/00*** *(2006.01)*   ***G01S 15/89*** *(2006.01)*
***A61B 8/08*** *(2006.01)*

(21) Application number: **07789717.1**

(22) Date of filing: **19.06.2007**

(86) International application number:
**PCT/IB2007/052336**

(87) International publication number:
**WO 2007/148279 (27.12.2007 Gazette 2007/52)**

(54) **METHOD, APPARATUS AND COMPUTER PROGRAM FOR THREE-DIMENSIONAL ULTRASOUND IMAGING**

VERFAHREN, VORRICHTUNG UND COMPUTERPROGRAMM ZUR DREIDIMENSIONALEN ULTRASCHALLABBILDUNG

PROCEDE, APPAREIL ET PROGRAMME D'ORDINATEUR POUR IMAGERIE ULTRASONORE TRIDIMENSIONNELLE

(84) Designated Contracting States:
**AT BE BG CH CY CZ DE DK EE ES FI FR GB GR HU IE IS IT LI LT LU LV MC MT NL PL PT RO SE SI SK TR**

(30) Priority: **23.06.2006 EP 06300720**

(43) Date of publication of application:
**18.03.2009 Bulletin 2009/12**

(73) Proprietor: **Koninklijke Philips Electronics N.V.**
**5621 BA Eindhoven (NL)**

(72) Inventors:
• **SOLER, Pau**
**75008 Paris (FR)**
• **GERARD, Olivier**
**75008 Paris (FR)**
• **DELSO, Gaspar**
**75008 Paris (FR)**
• **ALLAIN, Pascal**
**75008 Paris (FR)**

(74) Representative: **Schouten, Marcus Maria**
**Philips**
**Intellectual Property & Standards**
**P.O. Box 220**
**5600 AE Eindhoven (NL)**

(56) References cited:
**US-A1- 2004 193 042**   **US-A1- 2005 228 280**
**US-A1- 2006 020 204**

## Description

### FIELD OF THE INVENTION

This invention relates to medical ultrasound imaging and, more particularly, to a method, an apparatus and a computer program for three-dimensional ultrasound imaging.

### BACKGROUND OF THE INVENTION

Three-dimensional echocardiology has many benefits in medical diagnosis in comparison to classical two-dimensional imaging. For instance, it allows more accurate quantification such as left ventricle volume measurement and ejection fraction computation, since no hypotheses are made on the shape of the left ventricle. Moreover, the overall examination time is reduced as most parts of the heart are visible within a single acquisition.

On the other hand, since the amount of data to be collected is larger than in the two-dimensional case, both spatial and temporal resolution are reduced. To obtain high-resolution large volumes, the acquisition of the left ventricle is usually divided in four sectors, each sector being obtained in a different cardiac cycle. However, this need of several cardiac cycles to obtain high-resolution volume may cause disjunctions between different sectors of the data if movement of heart is not exactly periodic during the several cardiac cycles.

As an example, medical ultrasound three-dimensional imaging system according to US 5,993,390 acquires ultrasound image data representative of three-dimensional sectors of a volume of interest in a patient in synchronism with corresponding cardiac cycles of the patient, and combines the image data representative of the sectors to provide image data representative of a three-dimensional ultrasound image of the volume.

As mentioned in this patent, a disadvantage of the system is that motion of the heart between heartbeats may cause discontinuities in the displayed image.

### SUMMARY OF THE INVENTION

An aim of this invention is to provide an acquisition method that allows avoiding junction artefacts in a three-dimensional image of a volume of interest.

According to the invention, a method according to claim 1 is provided.

Acquiring with a first, low resolution allows obtaining in a single cardiac cycle an image of the whole volume of interest. This image is used as a reference for the sectors acquired at the second, higher resolution. By comparing a sector acquired at high resolution with the corresponding portion of the volume acquired at low resolution, it can be assessed if the sector has moved or not between the acquisition of the volume and the acquisition of the sector. This information allows avoiding artifacts in the final high-resolution image, because a sector that has moved between the acquisition of the volume and the acquisition of the sector creates artifact in the final high-resolution image if it is not further processed.

The invention also relates to an apparatus for medical ultrasound imaging, as well as to a computer program for implementing the method in accordance with the invention.

### BRIEF DESCRIPTION OF THE DRAWINGS

The present invention will now be described in more detail, by way of example, with reference to the accompanying drawings, wherein :

Fig.1 is a cross-sectional view of a three-dimensional volume (Fig.1A) that is divided into sectors (Fig.1B);
Fig.2 is a schematic representation of a three-dimensional volume displaying artefacts;
Fig.3 shows an ECG waveform that is divided into twenty cardiac phases;
Fig.4 shows a flow diagram of acquisition of the images according to the invention.

### DETAILED DESCRIPTION

All of the known prior art three-dimensional cardiac imaging techniques have had low resolution and/or long acquisition times. In the case of long acquisition times, the images typically exhibit discontinuities due to cardiac, respiratory, patient and/or sonographer movement. As mentioned before, due to the need of high resolution, echocardiography full-volume acquisitions are currently performed within a plurality of cardiac cycles, for instance four. At each cycle, a sector of only one fraction (for instance one fourth) of the whole cardiac volume is imaged. The imaged cardio-vascular organ may also be only the left ventricle, but still it is imaged using four different sectors. It should be noticed that other interest organs may be imaged in accordance with the invention, as soon as their movement has a certain periodicity which is a function of the cardiac cycle.

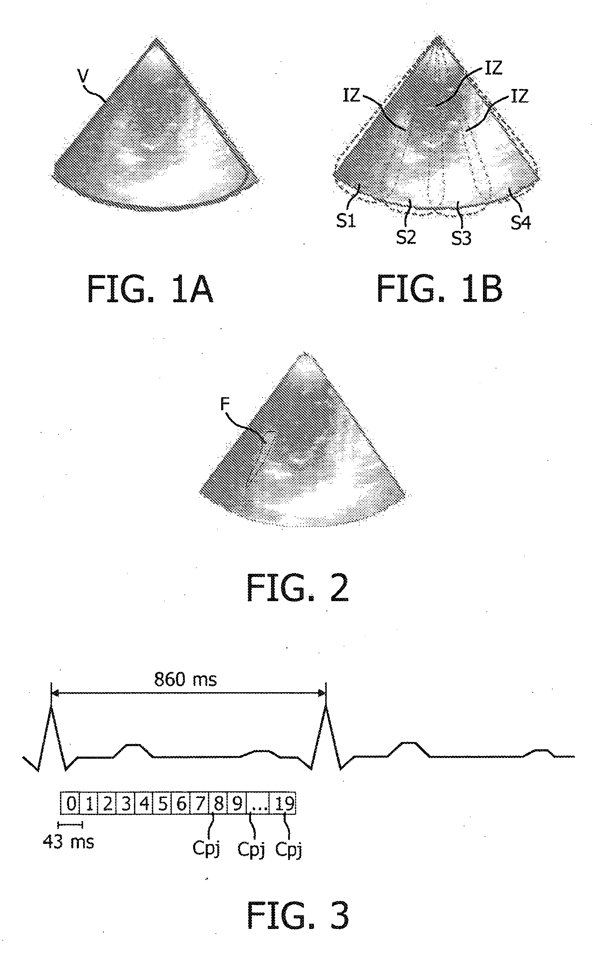

However, any movement of the patient (breath, displacement), of the ultrasound probe or any heart rate alteration (i.e. arrhythmia) results in a boundary between sectors and generate artefacts (F) as shown by arrow on Fig.2.

It should be noted that the closest prior art document US 2005/0228280 discloses a method for acquiring ultrasound data for display. The method comprises:

(a) scanning within a three-dimensional volume with a first spatial resolution; and
(b) scanning within a three-dimensional sub-volume of the volume with a second spatial resolution, the second spatial resolution being higher than the first spatial resolution.

In more details, step (b) comprises scanning within the entire three-dimensional sub-volume at the second spatial resolution and step (a) comprises scanning at the first spatial resolution within the entire three-dimensional volume other than the sub-volume.

A lower spatial resolution volume may be more rapidly scanned than a same region with a higher spatial resolution. By scanning the sub-volume with higher spatial resolution, medical diagnosis may be improved or based on more information content. However such medical diagnosis implies that the user have a sufficient medical knowledge to use the image. By scanning the remainder of the three-dimensional volume with a lower resolution, anatomical reference information at a lower resolution may be provided for helping the operator to position the sub-volume associated with higher resolution imaging. However, this document does not disclose that the image of a sub-volume acquired at high resolution is compared to the image of the same sub-volume acquired at lower resolution. In addition, the three-dimensional sub-volume and the three-dimensional volume other than said sub-volume refer to two distinct regions of the volume to be imaged and thus artefacts may occur at boundaries between said volume and sub-volume.

The invention described hereafter is an acquisition method to obtain large high-resolution image volumes without artefacts, the apparatus for implementing such method and the computer program to control the operation of the apparatus.

The apparatus for medical ultrasound imaging is defined in claim 9.

Additionally, other components may be provided, such as a memory for storage of ultrasound data, a display and a user input. The beamformer is operable to scan within a three-dimensional volume with one spatial resolution and operable to scan within a sub-volume or sector of the three-dimensional volume with a higher spatial resolution. For example, one or more of the frequency of scanning, imaging bandwidth, aperture size, aperture location, apodization type, scan geometry and scan line density are varied depending on which portion of a three-dimensional volume is being scanned. With higher frequency, a larger aperture and a denser scan line distribution, a sub-volume or sector is scanned with a higher spatial resolution. A lower spatial resolution volume may be more rapidly scanned than a same region with a higher spatial resolution.

The beamformer is operable to switch between parameters, such as aperture, frequency, apodization profile, delay profile and combinations thereof between transmit and receive events in order to vary a lateral extent, a scanning position, or resolution. By switching between beamforming parameters any of various combinations of scan patterns may be provided, such as scanning the entire three-dimensional volume in a first resolution mode, for example low resolution, during a first cardiac cycle and then scanning a sub-volume or sector with a second resolution mode, for instance higher resolution, during another cardiac cycle.

The apparatus may use a computer program which control the switching of the beamformer from one resolution mode to a second higher resolution mode, which synchronise the acquisition of the image data with the cardiac cycles signals delivered by, for example an ECG (stands for Electro Cardio Gram) sensor, and which performs the steps of the method in accordance with the invention.

The beamformer signals may be stored in an image data buffer which, as described below, stores image data for different volume sectors (VSi) of an image volume (V) illustrated on Figs.1A and 1B (in Fig.1B the sectors $VS_1$, $VS_2$, $VS_3$ and $VS_4$ are respectively named S1, S2, S3 and S4), and for different cardiac phases (CPj) of a cardiac cycle Ck illustrated on Fig.3.

A volume (V) may have a conical shape with an apex centred on the transducer array. A preferred application of the invention is cardiac imaging. To facilitate cardiac imaging, volume (V) may be divided into three-dimensioual sectors (VSi) for imaging of the patient's heart.

Thus, for example, the cross-sections of the sectors may be square, rectangular, circular, or irregularly shaped. Furthermore, different sectors may have different sizes and shapes within a single volume.

For a given volume, the selection of the size, shape and number of sectors may be based in part on the time available for image data acquisition during a specified cardiac phase as described below.

It will be understood that the volume itself is not limited to a conical shape and may have a variety of different shapes and sizes. For example, the volume may be a pyramid or a truncated pyramid. The selection of the size and shape of the volume may be based on the application and the type of transducer being utilized

A feature of the invention is based on acquisition of image data for one or more sectors in synchronism with the patient's cardiac cycle. An example of an ECG waveform is shown in Fig.3. In the example of Fig.3, ECG waveform indicates a heartbeat every 860 milliseconds. The cardiac cycle may be divided into cardiac phases for imaging. In one example, 20 cardiac phases CPj of approximately 43 milliseconds each may be utilized. The selection of the cardiac phase duration is typically based on the maximum time in which the heart does not move significantly. More or fewer cardiac phases may be utilized.

The ECG waveform of the patient is used to trigger image data acquisition, so that data acquisition is synchronized to each patient's cardiac cycle. More specifically, image data acquisition is synchronized to a specific phase of the cardiac cycle. Furthermore, image data may be acquired during each phase of each cardiac cycle. The amount of image data acquired during each cardiac phase is a function of the duration of the cardiac phase and the speed of image data acquisition

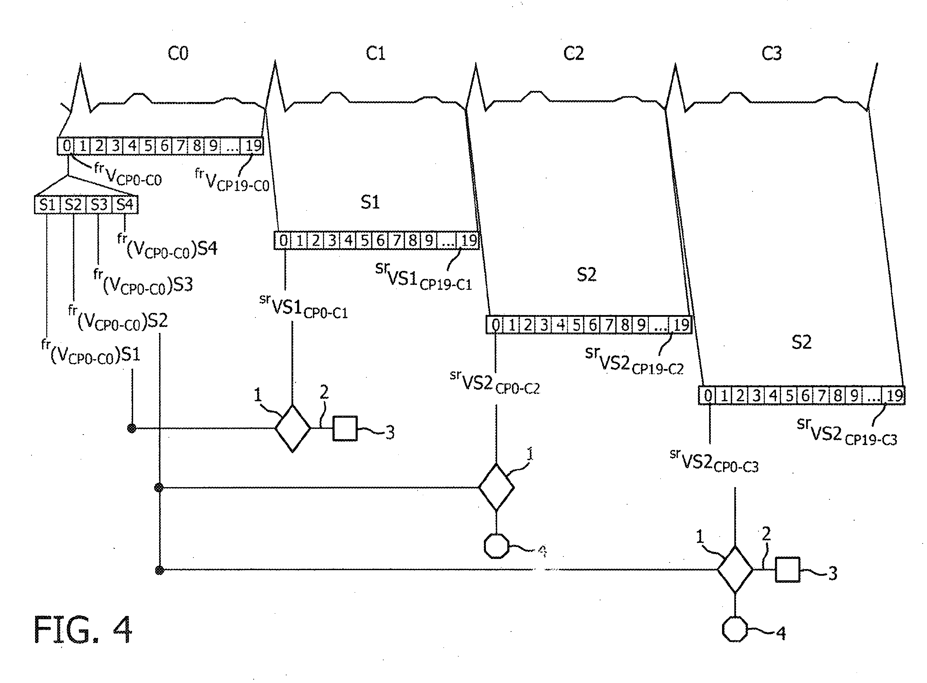

The acquisition of the image data for the number of sectors which constitute the volume is described with reference to FIG.4. The volume (V) is defined as having sectors (VS1-VS4). Each cardiac cycle Ck is defined as having cardiac phases (CP0-CP19). Image data is acquired during for example four cardiac cycles C0-C3. Using this notation, image data $^{fr}V_{CP0-C0}$ for volume (V) is acquired during cardiac phase CP0 of cardiac cycle C0 with a first resolution (fr). Image data $^{fr}V_{CP1-C0},......,^{fr}Y_{CP19-C0}$ for volume (V) are acquired during cardiac phases CP1-CP19 of cardiac cycle C0.

In the description $^{fr}(V_{CP1-C0})Si= FRsi$ designate the portion of data corresponding to sector (Si) and acquired for volume (V) with the first resolution during phases $CP_J$ of first cardiac cycle C0. Image data $^{sr}VS1_{CP0-C1}, ^{sr}VS1_{CP1-C1},.....,^{sr}VS1_{CP19-C1}$ for volume sector VS1 are similarly acquired with a second resolution (sr) during each of cardiac phases (CP0-CP19) of cardiac cycle C1. In the following $^{sr}VSi_{CPj-Ck}= SRi$ designate the image data of second resolution acquisition made on sector Si during cardiac phase (CPj) of cardiac cycle Ck.

According to the method of the invention shown on Fig.4, the first $^{fr}(V_{CP0-c0})Si$ and the second $^{sr}VSi_{CP0-C0}$ image data of the same sector Si are compared to a determined condition to validate the three-dimensional ultrasound second image data representative of the sector when the comparison satisfy this condition.

Each SR sector (SR$_i$) is compared (1) to the FR acquisition (FRsi). In one embodiment validation (2) of the different sectors is performed by calculating the similarity T$_i$ between the image data of the FR and the SR sectors, in a sum of squared differences sense.

$$T_i = \min_{T_i} \sum_{\forall x \in (FR \cap SR)} \left( FRsi(\mathbf{x}) - SR_i(\mathbf{x}) \right)^2$$

If the sum does not exceed a predetermined value the image data of second resolution are saved (3) in a memory of the apparatus or directly displayed on a display. If not a new acquisition (4) of the same sector is triggered at the beginning of the following cardiac cycle, until the image data satisfy the condition or until a given number of acquisition have been unsuccessful on the same sector and in this last case a warning signal is transmitted to the operator. A warning signal may also be outputted as soon as the above-mentioned sum exceeds the predetermined value. One advantage of the method is to obtain large high-resolution image volumes without artefacts.

As an example illustrated in Fig.4, if during the comparison between $(^{fr}V_{CP0-C0})S2$ and $^{sr}VS2_{CP0-C2}$ the above-mentioned sum exceeds the predetermined value the image data of second resolution obtained during cardiac cycle C2 are not saved (3) in a memory of the apparatus. A new acquisition is triggered (4) and realized during the following cardiac cycle C3, until the image data satisfy the condition, or a number of unsuccessful acquisitions have been attempted.

Further, if a given number of image data $^{sr}VSi_{CPj-Ck}$ acquired during the first phases of a cardiac cycle Ck are matching the condition and the remaining image data acquisitions during the remaining phases are not matching the condition, the system may interpolate these non-matching or invalidated acquisitions, thus avoiding to start a new acquisition for the remaining phases of the same sector during a following cardiac cycle. Actually, the image data matching the condition may be used to interpolate the image data not matching the condition. In other words, the image data not matching the condition may be re-calculated so as to be positioned at the same location than the image data matching the condition.

Alternatively, the image data not matching the condition may be re-calculated so as to position them differently until they reach the condition. In such a way, another acquisition is also avoided, the proper positioning of the sectors in the volume is realized by means of a re-calculation of the image data of each invalidated sector.

The said approach is used during a sufficient number of cardiac cycles, for instance six C0-C5. First cycle C0 is used for first resolution acquisition on the volume (V) and assuming for example that one volume sector (for example S2) needs a new acquisition, five other cycles C1-C5 are needed to acquire the four sectors (S1-S4), so that image data for the four volume sectors are acquired during each of cardiac phases CP0-CP19 of the cardiac cycles for delivering image data matching the condition.

Hence the present invention describes a robust acquisition protocol to overcome the potential junction artefacts originated in a full-volume acquisition process.

If the final difference between SR and FR image data exceeds a certain threshold, the operator may be warned that artefacts might be present in the acquisition. Moreover, a posteriori verification on the seamless transition from sector to sector can be performed by inspection of the gradient image or other similar low-level image processing algorithms.

According to an improvement of the invention, the sectors are chosen such that at least two sectors partially overlap. This situation is shown in Fig.1B. In case of no artifact, the image data in the overlapping regions should be the same for two partially overlapping sectors. As a consequence, if the image data in an overlapping region differs from one sector to another consecutive and overlapping sector, this means that there is an artifact and that said consecutive sector should be invalidated or further processed.

According to this improvement, comparison of the overlapping portions may be performed by calculating the similarity between the sectors, in a sum of squared differences sense, as of:

$$T_i = \min_{T_i} \sum_{\forall x \in (SR_i \cap SR_{i+1})} \left( SR_i(\mathbf{x}) - SR_{i+1}(\mathbf{x}) \right)^2$$

where $T_0$ is assumed to be the identity (the first sector serves as reference).

This improvement allows further improving reduction of the artefacts in the final image, as it may be used as an additional verification of the presence or absence of artefacts.

In another embodiment, the order of acquisition may also be important. If the FR acquisition is first performed, the SR sectors image data can be compared to it as long as they are acquired, without the need to wait until all acquisitions are finished. However, other acquisition orders are possible. If the FR volume is acquired in the middle, that is to say 2 SR acquisitions are performed, then the FR acquisition and then the remaining 2 SR acquisitions, the LR acquisition will be closer to all SR acquisitions, thus reducing the probability of artefacts in case there is a continuous drift in the acquisitions.

By obtaining a three-dimensional image representing the heart in each of the cardiac phases, a variety of information can be obtained. The three-dimensional images of the heart at successive cardiac phases can be displayed as a function of time to represent heart movement. The moving image can be used to identify end systole and end diastole and to perform other diagnostics. Images for a selected cardiac phase can be rotated to a desired orientation for improved analysis. Image analysis techniques can be utilized to quantify maximum and minimum volumes of the left ventricle. From this information, ejection volume and ejection fraction can be calculated.

The invention is not limited to a calculation of the similarity based on the sum of squared differences. Other similarity metrics could be used, such as the sum of the absolute differences, normalized correlation or normalized mutual information.

Any reference sign in the following claims should not be construed as limiting the claim. It will be obvious that the use of the verb "to comprise" and its conjugations does not exclude the presence of any other elements besides those defined in any claim. The word "a" or "an" preceding an element does not exclude the presence of a plurality of such elements.

**Claims**

1. A method for medical ultrasound imaging, comprising the steps of:

a) acquiring with a first resolution a first ultrasound image data representative of a volume of an interest organ during a cardiac cycle of a patient;

b) acquiring with a second resolution higher than the first resolution a second ultrasound image data representative of a three-dimensional sector of said volume during another cardiac cycle of the patient;

c) comparing the first and the second ultrasound image data of said sector so as to detect a similarity between the first and the second ultrasound image data and, as a function of said comparison,

- validating the three-dimensional ultrasound second image data if the first and the second ultrasound image data are substantially similar or otherwise
- further processing said sector.

2. A method for medical ultrasound imaging as claimed in claim 1 wherein the step of comparing the first and the second ultrasound image data comprises calculating their similarity according to one of a sum of squared differences, sum of the absolute differences, normalized correlation or normalized mutual information.

3. A method for medical ultrasound imaging as claimed in claim 1 wherein the step of validating the data comprises registering in a memory or displaying on a display the second ultrasound image data of the sector.

4. A method for medical ultrasound imaging as claimed in claim 1, wherein the step of further processing the sector comprises either acquiring a new second resolution ultrasound image data of said sector and iterating the comparing step or interpolating the three dimensional ultrasound second image data.

5. A method for medical ultrasound imaging as claimed in claim 1 wherein the volume is a whole patient's cardio-vascular organ and the method comprises acquiring with the second resolution a plurality of second ultrasound image data representative of a plurality of sectors during a plurality of cardiac cycles of the patient, said plurality of sectors covering the whole patient's cardio-vascular organ.

6. A method for medical ultrasound imaging as claimed in claim 5 wherein at least two sectors partially overlap.

7. A method for medical ultrasound imaging as claimed in claim 5 wherein the step of acquiring a volume or a sector comprises the acquisition of image data during several phases of a cardiac cycle and the step of comparing compares the image data of a sector acquired with a first resolution during a cardiac phase to the image data of the same sector acquired with a second resolution during the same cardiac phase

8. A method for medical ultrasound imaging as claimed in claim 1 wherein the step of further processing comprises a step of outputting a warning signal.

9. Apparatus for medical ultrasound imaging, comprising:

- an acquisition system arranged for :

- acquiring with a first resolution a first ultrasound image data representative of a volume of an interest organ during a cardiac cycle of a patient;
- acquiring with a second resolution higher than the first resolution a second ultrasound image data representative of a three-dimensional sector of said volume during another cardiac cycle of the patient; and

- a comparator for comparing the first and the second ultrasound image data of said sector so as to detect a similarity between the first and the second ultrasound image data, wherein the apparatus is adapted, as a function of said comparison, to validate the second ultrasound image data if the first and the second ultrasound image data are substantially similar or otherwise to further process said sector.

10. A computer program for controlling a medical ultrasound imaging apparatus, wherein the computer program performs the steps of:

- controlling the acquisition with a first resolution of a first ultrasound image data representative of a volume of an interest organ during a cardiac cycle of a patient;
- controlling the acquisition with a second resolution higher than the first resolution of a second ultrasound image data representative of a three-dimensional sector of said volume during another cardiac cycle of the patient; and
- comparing the first and the second ultrasound image data of said sector so as to detect a similarity between the first and the second ultrasound image data and as a function of said comparison, validating the second ultrasound image data representative of the sector if the first and the second ultrasound image data are substantially similar or otherwise processing further the sector.

**Patentansprüche**

1. Verfahren zur medizinischen Ultraschallbildgebung, das die folgenden Schritte umfasst:

a) Erfassen erster Ultraschallbilddaten, welche ein Volumen eines interessierenden Organs eines Patienten darstellen, während eines Herzzyklus eines Patienten mit einer ersten Auflösung;
b) Erfassen zweiter Ultraschallbilddaten, welche einen dreidimensionalen Sektor des genannten Volumens darstellen, während eines anderen Herzzyklus des Patienten mit einer zweiten Auflösung, die höher ist als die erste Auflösung;
c) Vergleichen der ersten und der zweiten Ultraschallbilddaten des genannten Sektors, um eine Ähnlichkeit zwischen den ersten und den zweiten Ultraschallbilddaten zu detektieren, und als eine Funktion des genannten Vergleichs

- Validieren der dreidimensionalen zweiten Ultraschallbilddaten, wenn die ersten und die zweiten Ultraschallbilddaten im Wesentlichen ähnlich sind, oder andernfalls
- den genannten Sektor weiter verarbeiten.

2. Verfahren zur medizinischen Ultraschallbildgebung nach Anspruch 1, wobei der Schritt des Vergleichens der ersten und der zweiten Ultraschallbilddaten das Berechnen ihrer Ähnlichkeit gemäß entweder einer Summe der quadrierten Differenzen, einer Summe der absoluten Differenzen, einer normalisierten Korrelation oder einer normalisierten wechselseitigen Information umfasst.

3. Verfahren zur medizinischen Ultraschallbildgebung nach Anspruch 1, wobei der Schritt des Validierens der Daten das Registrieren der zweiten Ultraschallbilddaten des Sektors in einem Speicher oder das Anzeigen dieser Daten auf einer Anzeigevorrichtung umfasst.

4. Verfahren zur medizinischen Ultraschallbildgebung nach Anspruch 1, wobei der Schritt des weiteren Verarbeitens des Sektors entweder das Erfassen neuer Ultraschallbilddaten des genannten Sektors mit einer zweiten Auflösung und die Iteration des Vergleichsschritts umfasst oder das Interpolieren der zweiten dreidimensionalen Ultraschallbilddaten.

5. Verfahren zur medizinischen Ultraschallbildgebung nach Anspruch 1, wobei das Volumen ein ganzes kardiovaskuläres Organ des Patienten ist und das Verfahren das Erfassen einer Vielzahl von zweiten Ultraschallbilddaten, die eine Vielzahl von Sektoren darstellen, während einer Vielzahl von Herzzyklen des Patienten mit der zweiten Auflösung umfasst, wobei die genannte Vielzahl von Sektoren das ganze kardiovaskuläre Organ des Patienten abdeckt.

6. Verfahren zur medizinischen Ultraschallbildgebung nach Anspruch 5, wobei sich mindestens zwei Sektoren teilweise überlappen.

7. Verfahren zur medizinischen Ultraschallbildgebung nach Anspruch 5, wobei der Schritt des Erfassens eines Volumens oder eines Sektors das Erfassen von Bilddaten während mehrerer Phasen eines Herzzyklus umfasst und der Schritt des Vergleichens die Bilddaten eines Sektors, die mit einer ersten Auflösung während einer Herzphase erfasst wurden, mit den Bilddaten des gleichen Sektors vergleicht, die während der gleichen Herzphase mit einer zweiten Auflösung erfasst wurden.

8. Verfahren zur medizinischen Ultraschallbildgebung nach Anspruch 1, wobei der Schritt des weiteren Verarbeitens einen Schritt des Ausgebens eines Warnsignals umfasst.

9. Gerät zur medizinischen Ultraschallbildgebung, das Folgendes umfasst:

   - ein Erfassungssystem, das ausgelegt ist zum:

     - Erfassen erster Ultraschallbilddaten, welche ein Volumen eines interessierenden Organs darstellen, während eines Herzzyklus eines Patienten mit einer ersten Auflösung;
     - Erfassen zweiter Ultraschallbilddaten, welche einen dreidimensionalen Sektor des genannten Volumens darstellen, während eines anderen Herzzyklus des Patienten mit einer zweiten Auflösung, die höher ist als die erste Auflösung; und

   - einen Komparator zum Vergleichen der ersten und der zweiten Ultraschallbilddaten des genannten Sektors, um eine Ähnlichkeit zwischen den ersten und den zweiten Ultraschallbilddaten zu detektieren, um als eine Funktion des genannten Vergleichs die zweiten Ultraschallbilddaten zu validieren, wenn die ersten und die zweiten Ultraschallbilddaten im Wesentlichen ähnlich sind, oder andernfalls den genannten Sektor weiter zu verarbeiten.

10. Computerprogramm zum Steuern eines medizinischen Ultraschallbildgebungsgeräts, wobei das Computerprogramm die folgenden Schritte durchführt:

    - Steuern der Erfassung erster Ultraschallbilddaten, welche ein Volumen eines interessierenden Organs darstellen, während eines Herzzyklus eines Patienten mit einer ersten Auflösung;
- Steuern der Erfassung zweiter Ultraschallbilddaten, welche einen dreidimensionalen Sektor des genannten Volumens darstellen, während eines anderen Herzzyklus des Patienten mit einer zweiten Auflösung, die höher ist als die erste Auflösung;
- Vergleichen der ersten und der zweiten Ultraschallbilddaten des genannten Sektors, um eine Ähnlichkeit zwischen den ersten und den zweiten Ultraschallbilddaten zu detektieren, und als eine Funktion des genannten Vergleichs Validieren der zweiten Ultraschallbilddaten, welche den Sektor darstellen, wenn die ersten und die zweiten Ultraschallbilddaten im Wesentlichen ähnlich sind, oder andernfalls den genannten Sektor weiter verarbeiten.

## Revendications

1. Procédé pour imagerie médicale par ultrasons, comprenant les étapes consistant à :

   a) acquérir, avec une première résolution, une première donnée image par ultrasons représentative d'un volume d'un organe d'intérêt au cours d'un cycle cardiaque d'un patient ;
   b) acquérir, avec une seconde résolution supérieure à la première résolution, une seconde donnée d'image par ultrasons représentative d'un secteur tridimensionnel dudit volume au cours d'un autre cycle cardiaque du patient ;
   c) comparer les première et seconde données image par ultrasons dudit secteur afin de détecter une similarité entre le première et seconde données image par ultrasons et, en fonction de ladite comparaison,

      - valider la seconde donnée image tridimensionnelle par ultrasons si les première et seconde données image par ultrasons sont sensiblement similaires ou autrement
      - traiter davantage ledit secteur.

2. Procédé pour imagerie médicale par ultrasons selon la revendication 1, dans lequel l'étape consistant à comparer les première et seconde données image par ultrasons comprend l'étape consistant à calculer leur similarité selon un élément parmi une somme de différences au carré, une somme de différences absolues, une corrélation normalisée ou des informations mutuelles normalisées.

3. Procédé pour imagerie médicale par ultrasons selon la revendication 1, dans lequel l'étape consistant à valider la donnée comprend l'étape consistant à enregistrer dans une mémoire ou afficher sur un affichage la seconde donnée image par ultrasons du secteur.

**4.** Procédé pour imagerie médicale par ultrasons selon la revendication 1, dans lequel l'étape consistant à traiter davantage le secteur comprend soit les étapes consistant à acquérir une nouvelle donnée image par ultrasons de seconde résolution dudit secteur et répéter l'étape consistant à comparer soit l'étape consistant à interpoler la seconde donnée image tridimensionnelle par ultrasons.

**5.** Procédé pour imagerie médicale par ultrasons selon la revendication 1, dans lequel le volume est un organe cardiovasculaire entier du patient et le procédé comprend l'étape consistant à acquérir, avec la seconde résolution, une pluralité de secondes données image par ultrasons représentatives d'une pluralité de secteurs au cours d'une pluralité de cycles cardiaques du patient, ladite pluralité de secteurs recouvrant l'organe cardiovasculaire entier du patient.

**6.** Procédé pour imagerie médicale par ultrasons selon la revendication 5, dans lequel au moins deux secteurs se chevauchent partiellement.

**7.** Procédé pour imagerie médicale par ultrasons selon la revendication 5, dans lequel l'étape consistant à acquérir un volume ou un secteur comprend l'acquisition de données image au cours de plusieurs phases d'un cycle cardiaque et l'étape consistant à comparer compare la donnée image d'un secteur acquise avec une première résolution au cours d'une phase cardiaque à la donnée image du même secteur acquise avec une seconde résolution au cours de la même phase cardiaque.

**8.** Procédé pour imagerie médicale par ultrasons selon la revendication 1, dans lequel l'étape consistant à traiter davantage comprend une étape consistant à produire un signal d'avertissement.

**9.** Appareil pour imagerie médicale par ultrasons, comprenant :

- un système d'acquisition agencé pour :

- acquérir, avec une première résolution, une première donnée image par ultrasons représentative d'un volume d'un organe d'intérêt au cours d'un cycle cardiaque d'un patient ;
- acquérir, avec une seconde résolution supérieure à la première résolution, une seconde donnée image par ultrasons représentative d'un secteur tridimensionnel dudit volume au cours d'un autre cycle cardiaque du patient ; et

- un comparateur pour comparer les première et seconde données image par ultrasons dudit secteur afin de détecter une similarité entre les première et seconde données image par ultrasons, dans lequel l'appareil est adapté, en fonction de ladite comparaison, pour valider la seconde donnée image par ultrasons si les première et seconde données image par ultrasons sont sensiblement similaires ou autrement pour traiter davantage ledit secteur.

**10.** Programme informatique pour commander un appareil d'imagerie médicale par ultrasons, dans lequel le programme informatique réalise les étapes consistant à :

- commander l'acquisition, avec une première résolution, d'une première donnée image par ultrasons représentative d'un volume d'un organe d'intérêt au cours d'un cycle cardiaque d'un patient ;
- commander l'acquisition, avec une seconde résolution supérieure à la première résolution, d'une seconde donnée image par ultrasons représentative d'un secteur tridimensionnel dudit volume au cours d'un autre cycle cardiaque du patient ; et
- comparer les première et seconde données image par ultrasons dudit secteur afin de détecter une similarité entre les première et seconde données image par ultrasons et, en fonction de ladite comparaison, valider la seconde donnée image par ultrasons représentative du secteur si les première et seconde données image par ultrasons sont sensiblement similaires ou autrement traiter davantage le secteur.

**FIG. 1A**

**FIG. 1B**

**FIG. 2**

**FIG. 3**

C0

C1

C2

C3

| 0 | 1 | 2 | 3 | 4 | 5 | 6 | 7 | 8 | 9 | ... | 19 |

$^{fr}V_{CP0\text{-}C0}$

$^{fr}V_{CP19\text{-}C0}$

| S1 | S2 | S3 | S4 |

$^{fr}(V_{CP0\text{-}C0})S4$

$^{fr}(V_{CP0\text{-}C0})S3$

$^{fr}(V_{CP0\text{-}C0})S2$

$^{fr}(V_{CP0\text{-}C0})S1$

S1

| 0 | 1 | 2 | 3 | 4 | 5 | 6 | 7 | 8 | 9 | ... | 19 |

$^{sr}VS1_{CP19\text{-}C1}$

$^{sr}VS1_{CP0\text{-}C1}$

S2

| 0 | 1 | 2 | 3 | 4 | 5 | 6 | 7 | 8 | 9 | ... | 19 |

$^{sr}VS2_{CP19\text{-}C2}$

$^{sr}VS2_{CP0\text{-}C2}$

S2

| 0 | 1 | 2 | 3 | 4 | 5 | 6 | 7 | 8 | 9 | ... | 19 |

$^{sr}VS2_{CP19\text{-}C3}$

$^{sr}VS2_{CP0\text{-}C3}$

1   2

3

1

4

1   2

3

4

# FIG. 4

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5993390 A

- US 20050228280 A